# EUROPEAN PATENT SPECIFICATION

(11) **EP 1 272 180 B1**
(45) Date of publication and mention of the grant of the patent: **29.09.2004**
(21) Application number: 01938050.0
(22) Date of filing: 27.03.2001
(51) Int. Cl.: A61K 31/403, A61K 9/48, A61K 47/12, A61K 47/14, A61P 9/00

(54) **CONCENTRATED SOLUTIONS OF CARVEDILOL**
KONZENTRIERTE LÖSUNGEN VON CARVEDILOL
SOLUTIONS CONCENTREES DE CARVEDILOL

(30) Priority: 03.04.2000 EP 00107094
(43) Date of publication of application: 08.01.2003
(73) Proprietor: F. HOFFMANN-LA ROCHE AG, 4070 Basel (CH)
(72) Inventor: DECKER, Silke, 65582 Diez (DE); GABEL, Rolf-Dieter, 68723 Schwetzingen (DE); LAPOTNIKOFF, Juergen, 74927 Eschelbronn (DE); WIRL, Alexander, 67259 Heuchelheim (DE); ZIMMERMANN, Ingfried, 97265 Hettstadt (DE)
(74) Representative: Brodbeck, Michel
(86) International application number: PCT/EP2001/003423
(87) International publication number: WO 2001/074356

(56) References cited:
- EP-A- 0 893 440
- WO-A-00/00179
- DE-A- 19 833 119

## Description

The present invention is concerned with concentrated pharmaceutically acceptable solutions of carvedilol and/or of a pharmaceutically acceptable salt thereof in adjuvants with predominantly lipophilic character, pharmaceutical administration forms containing such formulations as well as their use for the treatment or prophylaxis of illnesses.

Carvedilol is a non-selective β-blocker with a vasodilating component, which is brought about by antagonism to the α-adrenoreceptors. Moreover, carvedilol also has antioxidative properties. Carvedilol (1-(4-carbazolyloxy)-3-[2-(2-methoxyphenoxy)ethylamino]-2-propanol) is the object of European Patent No. 0 004 920 and can be manufactured according to the process described there.

"Pharmaceutically acceptable salts" of carvedilol embrace alkali metal salts, such as Na or K salts, alkaline earth metal salts, such as Ca and Mg salts, as well as salts with organic or inorganic acids, such as, for example, hydrochloric acid, hydrobromic acid, nitric acid, sulphuric acid, phosphoric acid, citric acid, formic acid, maleic acid, acetic acid, succinic acid, tartaric acid, methanesulphonic acid or toluenesulphonic acid, which are non-toxic for living organisms.

At pH values in the pharmaceutically relevant range of 1 to 8 the solubility of carvedilol in aqueous media lies between about 1 mg and 100 mg per 100 ml (depending on the pH value). This has been found to be problematical especially in the production of highly concentrated parenteral formulations, such as e.g. injection solutions or other formulations for use in small volume administration forms for ocular or oral administration.

In the case of the peroral administration of rapid release carvedilol formulations, e.g. the commercial formulation, resorption quotas of up to 80% are achieved, with a considerable part of the resorbed carvedilol being rapidly metabolized.

In connection with investigations into the gastrointestinal resorption of carvedilol it has been established that the resorption of carvedilol becomes poorer during the course of passage through the gastrointestinal tract and e.g. in the ileum and colon makes up only a fraction of the resorption in the stomach. This has been found to be very troublesome especially in the development of retard forms in which a release should take place over several hours. The poorer resorption is presumably due entirely or at least in part to the decreasing solubility of carvedilol with increasing pH values. A very low solubility can also be established in the strongly acidic region (about pH 1-2).

In order to improve the resorption quota, especially in the lower regions of the intestine, investigations have been carried out for adjuvants and, respectively, formulations which are suitable for increasing the solubility and/or speed of dissolution of carvedilol.

Accordingly, the underlying purpose of the invention lay in the provision of pharmaceutically acceptable, concentrated solutions of carvedilol suitable for further processing to carvedilol formulations with improved resorption properties, such as, for example, a modified release characteristic.

Thereby, it has surprisingly been found that it is not the combination with strong or medium strength organic or inorganic acids by salt formation which leads to the highest solubility improvements, but on the contrary the combination with quite particular adjuvants with lipophilic character. Thus, e.g. a slight increase in the solubility in aqueous media can be achieved by a combination with certain weak carboxylic acids, such as, for example, succinic acid, adipic acid, citric acid, tartaric acid, etc; however, certain adjuvants with lipophilic character lead to clearly higher increases in the solubility.

Under adjuvants with lipophilic character there are to be understood in connection with the present invention especially adjuvants selected from the group consisting of carboxylic acids, especially those with a long lipophilic molecular residue, organic alcohols, especially those with a lipophilic molecular component, and macrogol glycerol fatty acid esters. These adjuvants with lipophilic character can be used individually or in a combination of two or more adjuvants with one another.

It is especially surprising that the solubility of carvedilol in individual, specific carboxylic acids, especially those with a long lipophilic molecular residue, exhibit the best results with spacing. In the case of the saturated fatty acids there are to be named, for example, caproic acid, caprylic acid, capric acid, lauric acid and myristic acid. Preferred saturated fatty acids in connection with the present invention are caprylic acid, capric'acid and lauric acid, with capric acid being especially preferred. Preferred unsaturated fatty acids in connection with the present invention are linoleic acid, oleic acid, palmitoleic acid and erucic acid, with oleic acid being especially preferred. In the especially preferred carboxylic acids, oleic acid and capric acid, the solubility of carvedilol in each case amounts to > 100 mg/ml at 37°C.

Furthermore, it has been observed that certain organic alcohols can likewise bring about such a clear improvement. This is again especially true when a lipophilic molecular component is present. Benzyl alcohol, with a solubility of likewise > 100 mg of carvedilol per ml at 37°C, has been found to be especially favourable in this connection. Further, fatty alcohols, such as octanol, decanol, dodecanol, tetradecanol and hexadecanol, are also suitable.

Similar observations have also been made with macrogol glycerol fatty acid esters, especially macrogol glycerol laurate (Gelucire® 44/14) and macrogol glycerol stearate (Gelucire® 50/13). Diethylene glycol monoethyl ether (Transcutol® P) also has similarly good dissolving properties for carvedilol.

Especially preferred lipophilic adjuvants are oleic acid, capric acid, benzyl alcohol, macrogol glycerol laurate, macrogol glycerol stearate and diethylene glycol monoethyl ether.

These findings are surprising and new inasmuch as only clearly lower solubilities of carvedilol have been found with the solvents and, respectively, adjuvants usually used for the production of highly concentrated solutions of substances which are poorly soluble in water. Thus, the solubility of carvedilol in glycerol oleyloleate, medium chain triglycerides and natural oils, such as cod liver oil, soya bean oil, sesame oil, peanut oil, olive oil and cotttonseed oil, lies at blelow 50 mg/ml and in many cases at even below 10 mg/ml. Also, the solubility is very low in pronounced lipophilic solvents, such as paraffin, petroleum ether, etc.

Presumably, the molecules such as oleic acid, benzyl alcohol, macrogol glycerol laurate, macrogol glycerol stearate and diethylene glycol monoethyl ether, which have been ascertained to be especially favourable, have, inter alia, a quite specific ratio of suitable hydrophilic and lipophilic molecular components. This is especially evident in that e.g. organic carboxylic acids each with hydrocarbon chains which are only slightly shorter or, respectively, longer already fall off appreciably with respect to the dissolution properties for carvedilol. Apparently, e.g. the double bond in the - longer chain - oleic acid provides a relationship favourable for the solution of carvedilol similar to that as the shorter hydrocarbon chain in capric acid.

The present invention is accordingly concerned with pharmaceutically acceptable solutions of carvedilol or of a pharmaceutically acceptable salt thereof containing adjuvants with lipophilic character, with the carvedilol content lying above 5% (wt./wt., based on the solution) and the carvedilol being distributed in the solution as a molecular dispersion.

Under a molecular dispersion there is to be understood a monomolecular distribution of the active substance in a suitable carrier.

The carvedilol content in the solutions in accordance with the invention preferably lies at 5% (wt./wt., based on the solution) to 60% (wt./wt., based on the solution). In a further preferred embodiment the carvedilol content lies at 5% (wt./wt., based on the solution) to 50% (wt./wt., based on the solution). In an especially preferred embodiment the carvedilol content lies at 10% (wt./wt., based on the solution) to 40% (wt./wt., based on the solution).

Especially preferred are pharmaceutically acceptable solutions of carvedilol in oleic acid or Gelucire® (44/14), preferably Gelucire® (44/14), with the carvedilol content amounting to 5% (wt./wt., based on the solution) or above and the carvedilol being distributed in the solutiton as a molecular dispersion.

By a combination of carvedilol with the aforementioned adjuvants there can be made available concentrated solutions of carvedilol which permit the production of particular medicaments either generally for the first time or provide medicaments with especially advantageous properties. Thus, the solutions in accordance with the invention are especially suitable for the production of rapid release formulations or formulations with a modified release characteristic, such as, for example, retard forms with delayed release.

A rapid release formulation in accordance with the present invention is characterized in that about 95% of the active substance is released within about 2 hours, preferably about 50% of the active substance is released within 30 minutes. Under a modified release characteristic there is to be understood a 95% release after more than two hours, preferably after 2 to 24 hours, or a pH-dependent release in which the beginning of the release is delayed in time.

Thus, for example, there can be produced rapid release medicaments for peroral administration which exhibit an improved resorption over comparable medicaments which have been produced using crystalline carvedilol. In this case, formulations in accordance with the invention which contain oleic acid or Gelucire® (44/14) as the adjuvant are preeminently suitable for the production of such rapid release medicaments.

Furthermore, peroral forms with modified release, which in comparison to conventional medicaments produced using crystalline carvedilol exhibit clearly improved resorption properties, especially in the lower regions of the gastrointestinal tract, can be produced from the formulations in accordance with the invention. To these there belong e.g. forms which are resistant to gastric juice as well as retard forms in which the release extends over a long period. Thus, for example, peroral administration forms with the release characteristic of a product resistant to gastric juice can be produced in which no release takes place at pH 1 within 2 hours and a 95% release takes place at pH 6-8 within 2 hours.

For the production of such medicaments, the carvedilol solutions in the adjuvants in accordance with the invention can be used either directly or in combination with further additives (such as, for example, gel formers, antioxidants such as ascorbic acid and its derivatives or tocopherol and its derivatives) or in further processed form. The solutions in accordance with the invention can also be filled into pharmaceutically usable capsules, especially hydroxypropylmethylcellulose, hard gelatin and soft gelatin capsules, whereby the capsules can be modified in their pharmaceutically relevant behaviour (e.g. release behaviour) in a suitable manner, e.g. by coating. Thus, for example, the release of the active substance can be controlled by a coating which is resistant to gastric juice.

The concentrated carvedilol solutions in accordance with the invention and the medicaments produced therefrom can contain further additives, such as, for example, binders, plasticizers, diluents, carrier substances, glidants, antistatics, antioxidants, adsorption agents, separation agents, dispersants, dragéeing laquer, de-foamers, film formers, emulsifiers, extenders and fillers. When used in liquid form, there can be present, for example, flavour improving additives as well as substances usually used as preserving, stabilizing, moisture retaining and emulsifying agents as well as buffers and other additives.

The aforementioned additives can consist of organic or inorganic substances, e.g. water, sugar, salts, acids, bases, alcohols, organic polymeric compounds and the like. Preferred additives are lactose, saccharose, magnesium stearate, various celluloses and substituted celluloses, polymeric cellulose compounds, highly dispersed silicon dioxide, maize starch, talc, various polymeric polyvinylpyrrolidone compounds as well as polyvinyl alcohols arid their derivatives. It is a prerequisite that all additives used in the production are non-toxic and advantageously do not change the bioavailability of the active substance.

The liberation and, respectively, the resorption of the carvedilol from the solutions in accordance with the invention can be controlled by these additives. This is possible, for example, by the production of gels, especially oleogels, using pharmaceutically usual additives. Under an "oleogel" there is thereby to be understood a disperse system from at least two components, with a lipophilic liquid component being present in a gel former, such as e.g. hydrophobized silicon dioxide. Suitable additives which come into consideration are, for example, cellulose derivatives (such as hydroxypropylmethylcellulose (HPMC), hydroxypropylcellulose (HPC) etc.), colloidal silicon dioxide and its derivatives, bentonite (a water-containing layered silicate), polymethacrylates or other suitable organic or inorganic oleogel formers.

Thus, for example, pharmaceutical administration forms can be produced in which the active substance is released by erosion of the active substance-containing gel and/or diffusion from the gel matrix (e.g. carvedilol, adjuvant (e.g. oleic acid) and 10% gel former). In this manner there can be obtained systems in which the active substance release can be controlled by erosion and/or diffusion of the active substance from a matrix. The gel-like formulations can either be filled into capsules or (with appropriate consistency) be converted into solid administration forms in another suitable manner, such as, for example, by extrusion (e.g. by means of a worm extruder with subsequent rounding off to pellets) or spray solidification.

In the case of spray solidification, the material to be solidified is sprayed as a melt at the upper end of a wide, cylindrical container through a heatable atomizer arrangement to give a droplet mist. The resulting droplet mist is mixed with cooled air (preferably < 25°C), which is conducted into the dryer around the atomization zone. The heat of solidification which results is taken up by the air and transported away and the separated solidified powder is removed from the container via a separator. As atomizer arrangements there come into consideration (heatable) rotary pressure nozzles, pneumatic nozzles (binary/ternary nozzles) or centrifugal atomizers.

In a further variant the active substance can be dissolved in a warmed lipophilic or amphiphilic solvent and subsequently solidified, e.g. by means of spray solidification with an appropriate adjuvant (for example hydrophobized silicon dioxide).

Further, the use of the solutions in accordance with the invention or formulations therefrom in systems with osmotically controlled release is possible. In this case, the continuous release of medicament is dependent on osmotic forces. Water penetrates e.g. through a membrane into the reservoir with a swellable adjuvant which then as a result of an increase in volume forces out the medicament solution through a release opening.

The conversion of the carvedilol solutions in accordance with the invention into solid, flowable formulations is also an object of the Application. This can be effected e.g. by embedding the carvedilol solutions in accordance with the invention in other pharmaceutically usual adjuvants or adsorbing them on these. An example of this is the spray drying - where necessary at an elevated temperature - of an aqueous solution of hydroxypropylmethylcellulose in which the carvedilol solution in accordance with the invention is present in finely dispersed form. After the spray drying there is present a'solid, flowable product in which the carvedilol solution is present in finely dispersed form in or adsorbed on the hydroxypropylmethylcellulose (HPMC) particles. The conversion of the carvedilol solutions in accordance with the invention into solid, flowable formulations facilitates the production of solid administration forms of the formulations in accordance with the invention with a modified release characteristic.

In the case of spray drying, the material to be dried is sprayed as a solution or suspension at the upper end of a wide, cylindrical container through an atomizer arrangement to give a droplet mist. The resulting droplet mist is mixed with hot air (preferably > 100°C) or an inert gas which is conducted into the dryer around the atomization zone. The resulting solvent vapour is taken up by the drying air and transported away and the separated powder is removed from the container via a separator.

Finally, medicaments for dermal/transdermal application and for use on mucous membranes as well as forms for the parenteral administration of highly concentrated carvedilol formulations, which contain the carvedilol solutions in accordance with the invention with or without further additives (for example, solvents such as propylene glycol, ethanol, glycerol, water, polyethylene glycol, etc.; antioxidants such as ascorbic acid and its derivatives; buffers such as phosphate buffer, acetate buffer, citrate buffer; preservatives such as parabens; and/or salts for varying the osmotic pressure, such as sodium chloride), can also be produced from the formulations in accordance with the invention.

As pharmaceutically acceptable administration forms of the carvedilol formulations in accordance with the invention there come into consideration, for example, capsules, tablets, pellets and solutions. Capsules and tablets are preferred pharmaceutically acceptable administration forms.

Pharmaceutical administration forms containing the carvedilol solutions in accordance with the invention have an improved bioavailability compared with corresponding formulations containing crystalline carvedilol, since the active substance is resorbed more rapidly in dissolved form than in crystalline form.

The pharmaceutical solutions in accordance with the invention are suitable for the production of medicaments for the treatment and/or prophylaxis of cardiac and circulatory disorders, such as e.g. hypertension, cardiac insufficiency and angina pectoris.

The dosage in which the pharmaceutical formulations in accordance with the invention are administered depends on the age and the requirements of the patients and the route of administration. In general, dosages of about 1 mg to 50 mg of carvedilol come into consideration. For this, formulations with a carvedilol active substance content of about 1 mg to 50 mg are used.

The present invention is also concerned with a process for the production of pharmaceutically acceptable, concentrated carvedilol solutions, which comprises the admixture of carvedilol with suitable adjuvants with predominantly lipophilic character, such as, for example, capric acid, oleic acid, benzyl alcohol, diethylene glycol monoethyl ether, macrogol glycerol laurate or macrogol glycerol stearate or mixtures of these adjuvants with one another.

Further, the present invention is concerned with the use of said solutions for the manufacture of a medicament for the treatment of illnesses, such as hypertension, cardiac insufficiency or angina pectoris, which comprises the administration of medicaments which contain the pharmaceutical formulations described above.

The following Examples are intended to describe the preferred embodiments of the present invention, without thereupon limiting this.

### Example 1:

Carvedilol
Capric acid

The carvedilol is introduced into the capric acid, which is warmed to about 35°C, and stirred until solution is complete.

### Example 2:

Carvedilol
Oleic acid

The carvedilol is introduced portionwise into the oleic acid and stirred intensively under protection from light and under a N₂ atmosphere until the individual portions have dissolved. Alternatively, an acceleration of the dissolution of the active substance is possible in an ultrasound bath.

### Example 3:

Carvedilol
Gelucire® 44/14

The Gelucire® 44/14 (macrogol glycerol laurate), which is a wax-like solid at room temperature, is melted at about 50°C. The active substance is incorporated portionwise into the melt and stirred until solution is complete. Subsequently, the warm, liquid solution can be filled e.g. into HPMC capsules or processed further in another suitable manner. Alternatively, the solution can be left to cool and the solidified intermediate product can be further processed at a later point in time.

### Example A

Hard or soft gelatin capsules with the following composition can be produced with the carvedilol solutions in accordance with the invention:
Carvedilol
Oleic acid
Hard gelatin capsule

The carvedilol is introduced portionwise into the oleic acid and stirred intensively under protection from light and under a N₂ atmosphere until the individual portions have dissolved. Alternatively, an acceleration of the dissolution of the active substance is possible in an ultrasound bath. The carvedilol solution is subsequently filled into capsules. The capsules can be provided with an increased protection from leakage by banding or sticking the upper and lower parts in a suitable manner.

## Claims

1. A pharmaceutically acceptable solution of carvedilol or of a pharmaceutically acceptable salt thereof containing adjuvants with lipophilic character, wherein the carvedilol content lies above 5% wt./wt., based on the solution and the carvedilol is distributed in the solution as a molecular dispersion.

2. A pharmaceutically acceptable solution according to claim 1, wherein capric acid, oleic acid, benzyl alcohol, diethylene glycol monoethyl ether, macrogol glycerol laurate or macrogol glycerol stearate or mixtures of these adjuvants with one another is/are present as the adjuvant with lipophilic character.

3. A pharmaceutically acceptable solution according to claim 2, wherein oleic acid or macrogol glycerol laurate or mixtures of these adjuvants is/are present as the adjuvant.

4. A pharmaceutically acceptable solution according to claim 3, wherein macrogol glycerol laurate is present as the adjuvant.

5. A pharmaceutically acceptable solution according to any one of claims 1 to 4, wherein the carvedilol content amounts to 5% wt./wt., based on the solution to 60% wt./wt., based on the solution, preferably 5% wt./wt., based on the solution to 50% wt./wt., based on the solution, especially 10% wt./wt., based on the solution to 40% wt./wt., based on the solution.

6. A pharmaceutically acceptable administration form containing a pharmaceutically acceptable solution according to any one of claims 1 to 5.

7. A pharmaceutically acceptable administration form according to claim 6, which is a rapid release administration form.

8. A pharmaceutically acceptable administration form according to claim 6, wherein no release takes place at pH 1 within 2 hours and a 95% release takes place at pH 6 to 8 within 2 hours.

9. A pharmaceutically acceptable administration form according to claim 6, which has an active substance release extended in time in the range of 2 to 24 hours.

10. A pharmaceutically acceptable administration form according to claim 6, wherein the pharmaceutically acceptable solution according to any one of claims 1 to 5 is embedded in a gel matrix.

11. A pharmaceutically acceptable administration form according to claim 6, wherein the pharmaceutically acceptable solution according to any one of claims 1 to 5 is used in a system with osmotically controlled active substance release which is accepted for pharmaceutical use.

12. A pharmaceutically acceptable administration form according to any one of claims 6 to 11, which is an oral administration form.

13. The use of a pharmaceutically acceptable solution according to any one of claims 1 to 5 for the manufacture of a medicament for the treatment and/or prophylaxis of cardiac and circulatory disorders, such as hypertension, cardiac insufficiency or angina pectoris.

14. The use of a pharmaceutically acceptable solution according to any one of claims I to 5 for the production of medicaments for the treatment or prophylaxis of disorders, such as hypertension, cardiac insufficiency or angina pectoris.

15. A process for the production of a pharmaceutically acceptable solution according to any one of claims 1 to 5, which process comprises mixing carvedilol with the adjuvants to form a molecular dispersed solution.

16. A process according to claim 15, wherein the resulting solution is converted into a solid administration form by spray drying or spray solidification.

## Patentansprüche

1. Pharmazeutisch verträgliche Lösung von Carvedilol oder eines pharmazeutisch verträglichen Salzes davon enthaltend Hilfsstoffe mit lipophilem Charakter, wobei der Carvedilolgehalt über 5% Gew./Gew., bezogen auf die Lösung, liegt und das Carvedilol in der Lösung als eine molekulare Dispersion verteilt ist.

2. Pharmazeutisch verträgliche Lösung gemäß Anspruch 1, wobei Caprinsäure, Ölsäure, Benzylalkohol, Diethylenglycolmonoethylether, Macrogolglycerinlaurat oder Macrogolglycerinstearat oder Gemische dieser Hilfsstoffe mit einander als der Hilfsstoff mit lipophilem Charakter vorliegt/vorliegen.

3. Pharmazeutisch verträgliche Lösung gemäß Anspruch 2, wobei Ölsäure oder Macrogolglycerinlaurat oder Gemische dieser Hilfsstoffe als der Hilfsstoff vorliegt/vorliegen.

4. Pharmazeutisch verträgliche Lösung gemäß Anspruch 3, wobei Macrogolglycerinlaurat als der Hilfsstoff vorliegt.

5. Pharmazeutisch verträgliche Lösung gemäß einem der Ansprüche 1 bis 4, wobei der Carvedilolgehalt 5% Gew./Gew., bezogen auf die Lösung, bis 60% Gew./Gew., bezogen auf die Lösung, bevorzugt 5% Gew./Gew., bezogen auf die Lösung, bis 50% Gew./Gew., bezogen auf die Lösung, insbesondere 10% Gew./Gew., bezogen auf die Lösung, bis 40% Gew./Gew., bezogen auf die Lösung, beträgt.

6. Pharmazeutisch verträgliche Darreichungsform enthaltend eine pharmazeutisch verträgliche Lösung gemäß einem der Ansprüche 1 bis 5.

7. Pharmazeutisch verträgliche Darreichungsform gemäß Anspruch 6, welche eine Darreichungsform mit rascher Freisetzung ist.

8. Pharmazeutisch verträgliche Darreichungsform gemäß Anspruch 6, wobei keine Freisetzung bei pH 1 innerhalb von 2 Stunden stattfindet und eine 95%ige Freisetzung bei pH 6 bis 8 innerhalb von 2 Stunden stattfindet.

9. Pharmazeutisch verträgliche Darreichungsform gemäß Anspruch 6, welche eine in ihrer Dauer verlängerte Wirkstofffreisetzung in dem Bereich von 2 bis 24 Stunden hat.

10. Pharmazeutisch verträgliche Darreichungsform gemäß Anspruch 6, wobei die pharmazeutisch verträgliche Lösung gemäß einem der Ansprüche 1 bis 5 in einer Gelmatrix eingebettet ist.

11. Pharmazeutisch verträgliche Darreichungsform gemäß Anspruch 6, wobei die pharmazeutisch verträgliche Lösung gemäß einem der Ansprüche 1 bis 5 in einem System mit osmotisch kontrollierter Wirkstofffreisetzung verwendet wird, welche für den pharmazeutischen Gebrauch üblich ist.

12. Pharmazeutisch verträgliche Darreichungsform gemäß einem der Ansprüche 6 bis 11, welche eine orale Darreichungsform ist.

13. Verwendung einer pharmazeutisch verträglichen Lösung gemäß einem der Ansprüche 1 bis 5 zur Herstellung eines Medikaments zur Behandlung und/oder Prophylaxe von Herzund Kreislauferkrankungen, wie Hypertension, Herzinsuffizienz oder Angina Pectoris.

14. Verwendung einer pharmazeutisch verträglichen Lösung gemäß einem der Ansprüche 1 bis 5 zur Herstellung von Medikamenten zur Behandlung oder Prophylaxe von Erkrankungen wie Hypertension, Herzinsuffizienz oder Angina Pectoris.

15. Verfahren zur Herstellung einer pharmazeutisch verträglichen Lösung gemäß einem der Ansprüche 1 bis 5, wobei das Verfahren Mischen von Carvedilol mit den Hilfsstoffen, um eine molekulare dispergierte Lösung zu bilden, umfasst.

16. Verfahren gemäß Anspruch 15, wobei die sich ergebende Lösung durch Sprühtrocknung oder Sprüherstarrung in eine feste Darreichungsform umgewandelt wird.

## Revendications

1. Solution pharmaceutiquement acceptable de carvédilol ou d'un sel pharmaceutiquement acceptable de celui-ci contenant des adjuvants avec un caractère lipophile, dans laquelle la teneur en carvédilol se situe au-dessus de 5 % en poids, sur la base de la solution et le carvédilol est réparti dans la solution comme dispersion moléculaire.

2. Solution pharmaceutiquement acceptable selon la revendication 1, dans laquelle l'acide caprique, l'acide oléique, l'alcool benzylique, l'éther de diéthylène-glycol et de monoéthyle, le laurate de glycérol et de macrogol ou le stéarate de glycérol et de macrogol ou des mélanges de ces adjuvants les uns avec les autres est/sont présent(s) comme adjuvant avec un caractère lipophile.

3. Solution pharmaceutiquement acceptable selon la revendication 2, dans laquelle l'acide oléique ou le laurate de glycérol et de macrogol ou des mélanges de ces adjuvants est/sont présent(s) comme adjuvant.

4. Solution pharmaceutiquement acceptable selon la revendication 3, dans laquelle le laurate de glycérol et de macrogol est présent comme adjuvant.

5. Solution pharmaceutiquement acceptable selon l'une quelconque des revendications 1 à 4, dans laquelle la teneur en carvédilol atteint de 5 % en poids, sur la base de la solution, à 60 % en poids, sur la base de la solution, de préférence de 5 % en poids, sur la base de la solution, à 50 % en poids, sur la base de la solution, en particulier de 10 % en poids, sur la base de la solution, à 40 % en poids, sur la base de la solution.

6. Forme d'administration pharmaceutiquement acceptable contenant une solution pharmaceutiquement acceptable selon l'une quelconque des revendications 1 à 5.

7. Forme d'administration pharmaceutiquement acceptable selon la revendication 6, qui est une forme d'administration à libération rapide.

8. Forme d'administration pharmaceutiquement acceptable selon la revendication 6, dans laquelle aucune libération ne se produit à un pH de 1 dans les 2 heures et une libération à 95 % se produit à un pH de 6 à 8 dans les 2 heures.

9. Forme d'administration pharmaceutiquement acceptable selon la revendication 6, qui a une libération de substance active prolongée dans le temps dans une plage de 2 à 24 heures.

10. Forme d'administration pharmaceutiquement acceptable selon la revendication 6, dans laquelle la solution pharmaceutiquement acceptable selon l'une quelconque des revendications 1 à 5 est incluse dans une matrice de gel.

11. Forme d'administration pharmaceutiquement acceptable selon la revendication 6, dans laquelle la solution pharmaceutiquement acceptable selon l'une quelconque des revendications 1 à 5 est utilisée dans un système à libération de substance active contrôlée osmotiquement qui est accepté pour un usage pharmaceutique.

12. Forme d'administration pharmaceutiquement acceptable selon l'une quelconque des revendications 6 à 11, qui est une forme d'administration orale.

13. Utilisation d'une solution pharmaceutiquement acceptable selon l'une quelconque des revendications 1 à 5 pour la fabrication d'un médicament pour le traitement et/ou la prophylaxie de troubles cardiaques et circulatoires, tels que l'hypertension, l'insuffisance cardiaque ou l'angine de poitrine.

14. Utilisation d'une solution pharmaceutiquement acceptable selon l'une quelconque des revendications 1 à 5 pour la production de médicaments pour le traitement ou la prophylaxie de troubles, tels que l'hypertension, l'insuffisance cardiaque ou l'angine de poitrine.

15. Procédé de production d'une solution pharmaceutiquement acceptable selon l'une quelconque des revendications 1 à 5, lequel procédé comprend l'étape consistant à mélanger du carvédilol avec les adjuvants pour former une solution moléculaire dispersée.

16. Procédé selon la revendication 15, dans lequel la solution résultante est transformée en forme d'administration solide par séchage par pulvérisation ou solidification par pulvérisation.
